Europäisches Patentamt

European Patent Office

Office européen des brevets

(11) Publication number: **0 136 133**
**B1**

(12) **EUROPEAN PATENT SPECIFICATION**

(45) Date of publication of patent specification: **11.01.89**

(21) Application number: **84306186.2**

(22) Date of filing: **11.09.84**

(51) Int. Cl.⁴: **C 07 C 5/27,** C 07 C 15/08, B 01 J 29/06

(54) Xylene isomerization process.

(30) Priority: **28.09.83 US 536471**

(43) Date of publication of application:
**03.04.85 Bulletin 85/14**

(45) Publication of the grant of the patent:
**11.01.89 Bulletin 89/02**

(84) Designated Contracting States:
**BE DE FR GB IT NL**

(56) References cited:
**EP-A-0 008 871**
**EP-A-0 020 043**
**EP-A-0 039 205**
**EP-A-0 056 698**
**US-A-4 049 738**

(73) Proprietor: **MOBIL OIL CORPORATION**
**150 East 42nd Street**
**New York New York 10017 (US)**

(72) Inventor: **Kresge, Charles Theodore**
**140 Shire Drive**
**Sewell New Jersey 08080 (US)**
Inventor: **Nicoletti, Michael Peter**
**11 Fern Road**
**Turnersville New Jersey 08012 (US)**
Inventor: **Vartuli, James Clarke**
**320 Ponds Edge Road**
**West Chester Pennsylvania 19380 (US)**

(74) Representative: **Cooper, John Anthony et al**
**Mobil Court 3 Clements Inn**
**London WC2A 2EB (GB)**

Courier Press, Leamington Spa, England.

## EP 0 136 133 B1

**Description**

This invention relates to a xylene isomerization process.

Since the announcement of the first commercial installation of Octafining in Japan in June, 1958, this process has been widely installed for the supply of p-xylene. See "Advances in Petroleum Chemistry and Refining" volume 4, page 433 (Interscience Publishers, New York 1961). The demand for p-xylene has increased at remarkable rates, particularly because of the demand for terephthalic acid to be used in the manufacture of polyesters.

Typically, p-xylene is derived from mixtures of $C_8$ aromatics separated from such raw materials as petroleum naphthas, particularly reformates, usually by selective solvent extraction. The $C_8$ aromatics in such mixtures and their properties are:

|  | Freezing Point °F | Boiling Point °F |
|---|---|---|
| Ethylbenzene | -139.0 | 277.1 |
| P-xylene | 55.9 | 281.0 |
| M-xylene | -54.2 | 282.4 |
| O-xylene | -13.3 | 292.0 |

The $C_8$ aromatic fractions from these sources vary quite widely in composition but will usually be in the range 2 to 32 wt% ethylbenzene with the balance, xylenes, being divided approximately 50—65 wt% meta, and the balance para and ortho.

Individual isomer products may be separated from the naturally occurring mixtures by appropriate physical methods. Ethylbenzene may be separated by fractional distillation although this is a costly operation. Ortho-xylene may be selected by fractional distillation and is so produced commercially. Para-xylene is separated from the mixed isomers by fractional crystallization or by selective adsorption.

As commercial use of para and ortho-xylene has increased there has been interest in isomerizing the other $C_8$ aromatics toward an equilibrium mix and thus increasing yields of the desired xylenes. At present, several xylene isomerization processes are available and in commercial use.

The isomerization process operates in conjunction with the product xylene or xylenes separation process. A virgin $C_8$ aromatics mixture is fed to such a processing combination in which the residual isomers emerging from the product separation steps are then charged to the isomerizer unit and the effluent isomerizate $C_8$ aromatics are recycled to the product separation steps. The composition of isomerizer feed is then a function of the virgin $C_8$ aromatic feed, the product separation unit performance, and the isomerizer performance.

It will be apparent that separation techniques for recovery of one or more xylene isomers will not have material effect on the ethylbenzene introduced with charge to the recovery/isomerization "loop". Thus, ethylbenzene, normally present in eight carbon atom aromatic fractions, will accumulate in the loop unless excluded from the charge or converted by some reaction in the loop to products which are separable from xylenes by means tolerable in the loop. Ethylbenzene can be separated from the xylenes of boiling point near that of ethylbenzene by extremely expensive "superfractionation". This capital and operating expense cannot be tolerated in the loop where the high recycle rate would require an extremely large distillation unit for the purpose. It is a usual adjunct of low pressure, low temperature isomerization as a charge preparation facility in which ethylbenzene is separated from the virgin $C_8$ aromatic fraction before introduction to the loop.

Other isomerization processes operate at higher pressure and temperature, usually under hydrogen pressure in the presence of catalysts which convert ethylbenzene to products readily separated by relatively simple distillation in the loop, which distillation is needed in any event to separate by-products of xylene isomerization from the recycle stream. For example, the Octafining catalyst of platinum on a silica-alumina composite exhibits the dual functions of hydrogenation/dehydrogenation and isomerization.

In Octafining, ethylbenzene reacts through ethyl cyclohexane to dimethyl cyclohexanes which in turn equilibrate to xylenes. Competing reactions are disproportionation of ethylbenzene to benzene and diethylbenzene, hydrocracking of ethylbenzene to ethylene and benzene and hydrocracking of the alkyl cyclohexanes.

The rate of ethylbenzene approach to equilibrium concentration in a $C_8$ aromatic mixture is related to effective contact time. Hydrogen partial pressure has a very significant effect on ethylbenzene approach to equilibrium. Temperature change within the range of Octafining conditions 443°C to 482°C (830°F to 900°F) has but a very small effect on ethylbenzene approach to equilibrium.

Concurrent loss of ethylbenzene to other molecular weight products relates to percent approach to equilibrium. Products formed from ethylbenzene include $C_6+$ naphthenes, benzene from cracking, benzene and $C_{10}$ aromatics from disproportionation, and total loss to other than $C_8$ molecular weight. $C_5$ and lighter hydrocarbon by-products are also formed.

The three xylenes isomerize much more selectively than the reaction of ethylbenzene, but they exhibit

2

different rates of isomerization and hence, with different feed compositions the rates of approach to equilibrium vary considerably.

Ethylbenzene has been found to be responsible for a relatively rapid decline in catalyst activity and this effect is proportional to its concentration in a $C_8$ aromatic feed mixture. It has been possible then to relate catalyst stability (or loss in activity) to feed composition (ethylbenzene content and hydrogen recycle ratio) so that for any $C_8$ aromatic feed, desired xylene products can be made with a selected suitably long catalyst use cycle.

A different approach to conversion of ethylbenzene is described in U.S. patent 3,856,872, and is based on the discovery that in the presence of an active acid catalyst typified by zeolite ZSM—5, ethylbenzene disproportionates to benzene and diethylbenzene which are readily separated from xylenes by the distillation equipment needed in the loop to remove by-products. It is recognized that rate of disproportionation of ethylbenzene is related to the rate of conversion of xylenes to other compounds, e.g. by disproportionation. U.S. patent 3,856,873 also describes reaction of $C_8$ aromatics over ZSM—5 and shows the effect of varying the reaction temperature up to 510°C (950°F) in the absence of metal co-catalyst and in the absence of hydrogen.

In the known processes for accepting ethylbenzene to the loop, conversion of that compound is constrained by the need to hold conversion of xylenes to other compounds to an acceptable level. Thus, although the technique described in U.S. 3856872 provides significant advantages over Octafining in this respect, operating conditions are still selected to balance the advantages of ethylbenzene conversion against the disadvantages of xylene loss by disproportionation and the like.

U.S. Patent No. 4,163,028 discloses xylene isomerization and ethylbenzene conversion at high temperatures with ZSM—5 of very high silica to alumina ratio whereby the acid activity is reduced. U.S. 4,236,996 discloses xylene isomerization concurrently with ethylbenzene conversion utilizing a certain steamed ZSM—5 catalyst. An improved method for preparing PtZSM—5 and the use of such a catalyst in xylene isomerization is disclosed in U.S. 4,312,790.

The above recited prior art notwithstanding, there remain certain problems in the conversion of $C_8$ aromatics fractions which contain ethylbenzene. By their very nature, all of the proposed processes involve the high temperature isomerization of xylenes followed by reduction of temperature to crystallize paraxylene from the unwanted isomers, reheating of the recycle stream to high temperature, and repetition of this cycle exhaustively to convert xylenes to para-xylene. Thus, the processes occur at temperatures which are conducive to transalkylation reactions and the formation of transalkylation intermediates which ultimately results in xylene loss. In the past, efforts have been made to prevent the formation of the relatively bulky $C_9^+$ transalkylation intermediates and the resulting xylene loss by reducing the effective pore size of the ZSM—5 zeolite. U.S. Patent No. 3,906,054 discloses a method for reducing the effective pore size of ZSM—5 zeolite by incorporating a small amount of phosphorous in the crystal structure. However, such catalysts may suffer from diffusion of the phosphorus from the crystal structure, particularly if water is present in the feed.

It has now been found that formation of $C_9^+$ residue products from transalkylation reactions can be inhibited in xylene isomerizations even at temperatures greater than about 315°C (600°F) by employing ZSM—23 catalyst which has a slightly smaller pore diameter than ZSM—5 (4.5 × 5.6 A versus 4.8 × 7.1 A). Because of the reduced pore size of ZSM—23 relative to ZSM—5, the formation of transalkylation intermediates of undesired $C_9^+$ aromatics is reduced, thereby producing a significant increase in ethylbenzene selectivity. It has, however, also been found that in order to increase the xylene isomerization activity of the ZSM—23 it should be produced such that its pores are substantially unobstructed by silica, for example by using a non-gelling forming mixture which contains an amorphous precipitated silica.

Accordingly, the present invention resides in a process for isomerizing an isomerization feed containing an aromatic $C_8$ mixture of ethylbenzene and xylene by which the para-xylene content is enhanced, said process comprising contacting said feed under conversion conditions with a catalyst comprising ZSM—23 zeolite having pores which are substantially unobstructed by silica. In a particularly preferred embodiment the ZSM—23 zeolite is prepared from a non-gelling forming mixture containing amorphous precipitated silica as a silica source.

The xylene isomerization processing conditions preferably include a hydrogen to hydrocarbon ratio in the range of 0.1 to 10, and preferably 1 to 5, a temperature of from 260°C (500°F) to 538°C (1000°F), preferably in excess of 343°C (650°F), a pressure of 0 to 1500 psig (100—10450 kPa) and a weight hourly space velocity of between 0.5 and 100.

Zeolitic materials, both natural and synthetic, have been demonstrated in the past to have catalytic properties for various types of hydrocarbon conversion. Certain zeolitic materials are ordered, porous crystalline aluminosilicates having a definite crystalline structure as determined by X-ray diffraction, within which there are a large number of small cavities which may be interconnected by a number of still smaller channels or pores. These cavities and pores are uniform in size within a specific zeolitic material. Since the dimensions of these pores are such as to accept for adsorption molecules of certain dimensions while rejecting those of larger dimensions, these materials have come to be known as "molecular sieves" and are utilized in a variety of ways to take advantage of these properties.

Such molecular sieves, both natural and synthetic, include a wide variety of positive ion-containing crystalline materials such as aluminosilicates. These materials can be described as a rigid three-

dimensional framework of $XO_4$ and $YO_4$ wherein X is silicon and/or germanium, and Y is one or more of aluminum, gallium, iron, chromium, vanadium, molybdenum, arsenic, manganese, or boron. This framework is comprised of tetrahedra which are cross-linked by the sharing of oxygen atoms whereby the ratio of the total Y and X atoms to oxygen atoms is 1:2. The electrovalence of the tetrahedra containing aluminum is balanced by the inclusion in the crystal of a cation, for example an alkali metal or an alkaline earth metal cation. This can be expressed wherein the ratio of Y to the number of various cations, such as Ca/2, Sr/2, Na, K or Li, is equal to unity. One type of cation may be exchanged either entirely or partially with another type of cation utilizing ion exchange techniques in a conventional manner. By means of such cation exchange, it has been possible to vary the properties of a given zeolite by suitable selection of the cation. The spaces between the tetrahedra are occupied by molecules of water prior to dehydration.

The crystalline zeolite utilized herein is a member of a special class of zeolitic materials which exhibit unusual properties. Although these zeolites have unusually low Y atom contents, i.e. high X to Y mole ratios, e.g., high silica-alumina ratios, they are very active even when the X to Y mole ratio exceeds 30. The activity is surprising, since catalytic activity is generally attributed to framework Y atoms and/or cations associated with these atoms. These zeolites retain their crystallinity for long periods in spite of the presence of steam at high temperatures which induces irreversible collapse of the framework of other zeolites, e.g. of the X and A type. Also, when used as catalysts, these zeolites generally have low coke-forming activity and therefore are conducive to long times on stream between regenerations.

An important characteristic of the crystal structure of this particular class of zeolites is that it provides a selective constrained access to and egress from the intracrystalline free space by virtue of having an effective pore size intermediate between the small pore Linde A and the large pore Linde X, i.e., the pore windows of the structure are of about a size such as would be provided by 10-membered rings of silicon atoms interconnected by oxygen atoms.

The catalyst used herein is known as ZSM—23 and has a characteristic X-ray diffraction pattern, the values of which are set out in Table I, below.

## TABLE I

| d(A) | | | $I/I_0$ |
|---|---|---|---|
| 11.2 | ± | 0.23 | Medium |
| 10.1 | ± | 0.20 | Weak |
| 7.87 | ± | 0.15 | Weak |
| 5.59 | ± | 0.10 | Weak |
| 5.06 | ± | 0.10 | Weak |
| 4.50 | ± | 0.10 | Weak |
| 4.53 | ± | 0.10 | Strong |
| 3.50 | ± | 0.08 | Very Strong |
| 3.72 | ± | 0.08 | Very Strong |
| 3.62 | ± | 0.07 | Very Strong |
| 3.54 | ± | 0.07 | Medium |
| 3.44 | ± | 0.07 | Strong |
| 3.36 | ± | 0.07 | Weak |
| 3.16 | ± | 0.07 | Weak |
| 3.05 | ± | 0.06 | Weak |
| 2.99 | ± | 0.06 | Weak |
| 2.85 | ± | 0.06 | Weak |
| 2.54 | ± | 0.05 | Medium |
| 2.47 | ± | 0.05 | Weak |
| 2.40 | ± | 0.05 | Weak |
| 2.34 | ± | 0.05 | Weak |

These values were determined by standard techniques using a scintillation counter spectrometer with a strip chart pen recorder with the incident radiation being the K-alpha doublet of copper. The peak heights, I, and the positions as a function of 2 times theta, where theta is the Bragg angle, were read from the spectrometer chart. From these, the relative intensities, 100 $I/I_0$, where $I_0$ is the intensity of the strongest line or peak, and d (obs.), the interplanar spacing in Angstrom units, corresponding to the recorded lines, were calculated. It should be understood that this X-ray diffraction pattern is characteristic of all the species of ZSM—23. Ion exchange of the sodium ion with cations reveals substantially the same pattern with some minor shifts in interplanar spacing and variation in relative intensity. Other minor variations can occur depending on the silicon to aluminum ratio of the particular sample, as well as if it has previously been subjected to thermal treatment.

ZSM—23 can also be identified, in terms of mole ratios of oxides and in the anhydrous state, as follows:

(0.58—3.4) $M_{2/n}O:Y_2O_3:(-12XO_2)$ wherein M is at least one cation having a valence n, X is silicon and/or germanium, and Y is one or more of aluminum, gallium, iron, chromium, vanadium, molybdenum, arsenic, manganese or boron. A particularly preferred form of ZSM—23 is the aluminosilicate form wherein X is silicon and Y is aluminum.

In a preferred synthesized form, the zeolite has a formula, in terms of mole ratios of oxides and in the anhydrous state, as follows:

$$(0.7—2.8)R_2O:(0.08—0.25)M_2O:Y_2O_3:(50—220)XO_2$$

wherein R is a nitrogen-containing organic cation, such as, for example, that derived from pyrrolidine, M is an alkaline metal cation, especially sodium, and X and Y are as described above, particularly where X is silicon and Y is aluminum.

The original cation of the as-synthesized ZSM—23 is at least partly replaced in accordance with ion exchange techniques well known in the art by other cations to render the material catalytically active. Preferred replacing cations include hydrogen, rare earth metals, and metals of Groups IIA, IIIB, IVB, VIII, IB, IIB, IIIA, IVA.

In the present process, the ZSM—23 is preferably used in intimate combination with a hydrogenating component such as tungsten, vanadium, molybdenum, rhenium, nickel, cobalt, chromium, manganese, or most preferably a noble metal such as platinum or palladium where a hydrogenation-dehydrogenation function is to be performed. Combinations of the aforenoted metals may also be used. Such components can be exchanged or cocrystallized into the composition, impregated thereon or physically intimately admixed therewith. Such components can be impregnated in or on to ZSM—23 such as, for example, in the case of platinum, by treating the zeolite with a platinum metal-containing ion. Thus, suitable platinum compounds for this purpose include chloroplatinic acid, platinous chloride and various compounds containing the platinum amine complex.

The as-synthesized ZSM—23 should preferably be dehydrated at least partially prior to use in the present process. This can be done by thermal treatment, i.e. heating, to a temperature in the range of 50°C to 900°C in an inert temperature, such as air or nitrogen, and at atmospheric or subatmospheric pressures for between 1 and 48 hours. Dehydration can also be performed at lower temperature merely by placing the catalyst in a vacuum, but a longer time is required to obtain a sufficient amount of dehydration.

As described in detail in U.S. 4076842, ZSM—23 is conventionally prepared from an aqueous solution containing sources of an alkali metal oxide, preferably sodium oxide, sources of nitrogen-containing cation, preferably pyrrolidine, an oxide of Y as described above and an oxide of X as described above having a composition, in terms of mole ratios of oxides, falling within the following ranges:

$R^+/(R^+ + M^+)$: 0.25—0.95, preferably 0.40—0.70
$OH/SiO_2$: 0.01—0.5, preferably 0.03—0.2
$H_2O/OH$: 100—2000, preferably 200—600
$XO_2/Y_2O_3$: 12—1000, preferably 50—250 wherein R is an organic nitrogen-containing cation and M is an alkali metal ion, and maintaining the mixture until crystals of the zeolite are formed. (The quantity of $OH^-$ is calculated only from the inorganic sources of alkali without any organic base contribution). Thereafter, the crystals are separated from the liquid and recovered. Typical reaction conditions are set out below.

Temperature   121—204°C (250—400°F), preferably 149—191°C (300—375°F)
Time          10—200 hrs, preferably 16 to 100 hours

The crystalline product is dried, e.g. at 110°C (230°F), for from 8 to 24 hours. Of course, milder conditions may be employed if desired, e.g. room temperature under vacuum.

The composition for the synthesis of synthetic ZSM—23 can be prepared utilizing materials which can supply the appropriate oxide. Such compositions include aluminates, alumina, ammonia, silicates, silica hydrosol, silica gel, silicic acid and hydroxides. It will be understood that each oxide component utilized in the reaction mixture can be supplied by one or more essential reactants and they can be mixed together in any order. The reaction mixture can be prepared either batchwise or continuously. Crystal size and crystallization time of the resultant ZSM—23 composition will vary with the nature of the reaction mixture employed.

In U.S. 4076842, the silica source is colloidal silica which produces a gel when mixed with the other components of the starting mixture. However, while such conventionally prepared ZSM—23 generally exhibits sufficient selectivity to prevent excessive formation of undesired residues by transalkylation its activity for xylene isomerication is undesirably low. It is believed that pore obstruction by silica and/or silicon causes the reduced activity of the catalysts since it has been found that ZSM—23 whose pores are substantially unobstructed by silica exhibits significantly increased activity in xylene isomerization. Such ZSM—23 may be prepared from a non-gelling forming mixture which contains amorphous precipitated silica as the silica source and which preferably has a solids content greater than about 5 weight percent, more preferably 7 to 25 weight percent. The amorphous precipitated silica can be a synthetic wet-process, hydrated amorphous silica containing trace impurities of $Al_2O_3$ and NaCl and preferably having a particle size range of about 0.01 to 100 microns. Preferably, the particles are of a spherical shape with an average diameter of about 0.02 microns. These particles tend to agglomerate in loose "grape cluster" structures. Such precipitated silicas also generally have large surface area, typically ranging from 140 to 160 square meters per gram. Hi-Sil, a product of PPG Industries Chemical Division, FK—320; available from Degussa Corporation, QUSO from PQ Corporation, and ZEOFREE—80 manufactured by J. M. Huber Corporation, have all been found suitable for producing ZSM—23 having significantly reduced silica pore occlusion.

In addition to sources of silica, alkali metal and water, optional ingredients in the forming mixture include surfactants, soluble aluminum compounds such as alum $(Al_2(SO_4)_3)$, sodium chloride, as well as ZSM—23 seed crystals.

If desired, the ZSM—23 zeolite catalyst can be employed in combination with a support or binder material such as, for example, a porous inorganic oxide support or a clay binder. Non-limiting examples of such binder materials include alumina, zirconia, silica, magnesia, thoria, titania, boria and combinations thereof, generally in the form of dried inorganic oxide gels and gelatinous precipitates. Suitable clay materials include, by way of example, bentonite and kieselguhr. The relative proportion of crystalline aluminosilicate ZSM—23 to the total composition of catalyst and binder or support may vary widely with the ZSM—23 content ranging from between 1 to 99 percent by weight and more usually in the range of 5 to 80 percent by weight of the composition.

It is contemplated that any feedstock containing an aromatic $C_8$ mixture comprising ethylbenzene and ortho-, meta-, and para-xylene may be used as feed to the process of this invention. Generally, such mixture will have an ethylbenzene content in the range of 5—50 wt.%, an ortho-xylene content in the approximate range of 0—15 wt.%, and a meta-xylene content in the range of 0—70 wt.%. The feed may also contain non-aromatic hydrocarbons, i.e., naphthenes and paraffins. The present process is particularly useful in the processing of hydrocarbon xylene feeds which contain high concentrations of ethylbenzene, i.e., greater than about 10 weight percent, e.g., 30 to 50 weight percent.

The preferred ZSM—23 catalyst will contain from 0.01% to 1%, preferably 0.05% to .10% wt.% by weight of platinum to provide mild hydrogenation activity. The catalyst is prepared by conventional techniques including impregnation, base exchange, drying and air calcination. If desired, the activity of the catalyst can be modified by steaming for 1 or more hours at temperatures upwards of 149°C (300°F), the time, pressure and temperature being interrelated such that less time is required at higher temperatures and/or pressures.

The feed is contacted with the above-described catalyst at a temperature between 260°C (500°F) and 510°C (950°F); a weight hourly space velocity (WHSV) of 0.5 to 50, preferably 5 to 25, and a pressure of 0 to 1500 psig (100—10450 kPa), preferably between 20 and 400 psig (240—2860 kPa).

The reaction product effluent will contain ethane, benzene, toluene and other aromatic hydrocarbons with a high selectivity for para-xylene and with the formation of transalkylation $C_9^+$ aromatic by-products, such as trimethylbenzene, and ethylxylene being reduced.

The following Example will serve to illustrate the process of this invention.

## Example
### A — Preparation of Conventional HZSM—23 Catalyst

A silicate solution was prepared by mixing 52 parts colloidal silica (30% by weight) and 67 parts water. An aluminate solution was prepared by combining 9 parts water, 1 part sodium aluminate, 0.3 parts sodium hydroxide (50% by weight) and 5.5 parts pyrrolidine. These two solutions were combined with stirring in an autoclave. The reactants were mixed at room temperature for one hour. The autoclave was heated to 171°C (340°F) and maintained at this temperature for 72 hours. The resultant zeolite was then filtered, washed in a Buchner funnel and dried overnight at 121°C (250°F). The x-ray diffraction analysis indicated that the zeolite was ZSM—23 and the chemical analysis indicated that the silica to alumina molar ratio was 64.

### B — Preparation of HZSM—23 Catalyst from a Forming Mixture Comprising Amorphous Precipitated Silica as a Silica Source

A silica solution was prepared by mixing 14 parts HiSil 233 (90% silica by weight) and 54 parts water. An alumina solution was prepared by combining 31 parts water, 1 part aluminum sulfate, 0.9 parts sodium hydroxide (50% by weight) and 4.4 parts pyrrolidine. These two solutions were combined with stirring in an autoclave. The combined solution was mixed at room temperature for one hour. The autoclave was heated to 171°C (340°F) and maintained at that temperature for 88 hours. The resultant zeolite was then filtered, washed in a Buchner funnel and dried overnight at 121°C (250°F). X-ray diffraction analysis indicated that the zeolite was ZSM—23 and the chemical analysis indicated that the silica to alumina molar ratio was 72.

### C — Preparation of Platinum-Containing HZSM—23 Extrudate

A 65/35 HZSM—23/$Al_2O_3$ catalyst extrudate was prepared in the following manner. The zeolite produced in B above was mixed with gamma alumina to make a mixture of 65 parts zeolite and 35 parts alumina (by weight). Enough water was added to the mixture so that the resulting catalyst could be formed

into $\frac{1}{16}$" extrudates. These extrudates were activated by first calcining in nitrogen at 540°C (1000°F) followed by aqueous exchanges with ammonium nitrate solution and finally calcining in air at 540°C (1000°F).

5 g of the activated extruded catalyst were placed in a beaker. While the beaker was agitated, 2.95 ml of an aqueous solution of $[Pt(NH_3)_4]Cl_2$ containing the equivalent of 0.1 wt% Pt was added to the extrudate. The volume of Pt solution was determined by incipient wetness techhniques. The Pt-containing HZSM—23 extrudates were allowed to stand at ambient temperature for 6 hours and were then dried for 18 hours in a forced air draft at over 121°C (250°F). The dried extrudates were then calcined for one hour at 538°C (1000°F) in air.

D — Aromatics Screening Test Comparing Transalkylation Promoting Tendencies of Conventionally Prepared ZSM—23 and ZSM—23 Prepared from a Forming Mixture Containing Amorphous Precipitated Silica

The catalytic results presented in Table 1 were obtained from an aromatics screening test which follows the conversion of ethylbenzene and m-xylene. The formation of diethylbenzene and trimethylbenzene isomers from ethylbenzene and m-xylene, respectively, serves as an accurate indicator of the transalkylation promoting tendencies of the catalyst under investigation. The greater this tendency for transalkylation the higher the potential for xylene losses.

The test conditions were as follows:

> 1.0 g catalyst
> $H_2$ reduction at 399°C (750°F)
> Charge Stock: ethylbenzene or m-xylene
> Temperature: 399°C (750°F)
> WHSV: 10 hr$^{-1}$
> $H_2$/HC: 3
> Pressure: 0 psig (100 kPa)

Table 1 compares the screening test results obtained for the conventionally prepared ZSM—23 catalyst (A) will be ZSM—23 catalyst (B) prepared from amorphous precipitated silica. Both catalysts were in the acid form and did not contain platinum group metals. The test results indicate that Catalyst B had significantly greater activity for the conversion of both ethylbenzene and m-xylene than the conventionally prepared Catalyst A.

## Table 1

### Screening Test Results
#### ZSM-23 Activity Comparison

|  | Catalyst A | Catalyst B |
|---|---|---|
| Zeolite: $SiO_2/Al_2O_3$ molar ratio | 64 | 70 |
| Extrudate: Zeolite/Binder | 65/35 | 65/35 |
| Ethylbenzene Conversion (Wt %) | 4.81 | 9.57 |
| M-Xylene Conversion (Wt %) | 24.19 | 37.66 |

E — Aromatics Screening Test Comparing Transalkylation Promoting Tendencies of HZSM—5 Platinum-Containing Catalysts with HZSM—23 Platinum-Containing Catalyst

0.1 wt% platinum-containing HZSM—5 catalyst prepared in accordance with Example 5 of U.S. 4,312,790 was compared with the platinum-containing HZSM—23 catalyst B in a screening test under the same conditions used in Example D. The results of this comparison are given in Table 2. The screening test results indicate that under the same operating conditions, the ZSM—23 catalyst possessed a superior ability to convert both m-xylene and ethylbenzene with a reduced selectivity towards transalkylation and thus lower xylene losses.

7

Table 2

Screening Test Results

Transalkylation Activity Results

| Catalyst | ZSM-5 | Catalyst B |
|---|---|---|
| Ethylbenzene Conv., Wt % | 5.69** | 10.11 |
| Diethylbenzene, Wt % | | |
| Yield | 1.48 | 0.24 |
| Selectivity* | 26.01 | 2.37 |
| | | |
| M-Xylene Conv., Wt % | 32.91 | 36.53 |
| Trimethylbenzene, Wt % | | |
| Yield | 0.38 | 0.11 |
| Selectivity | 1.15 | 0.30 |

\* Selectivity $= \dfrac{\text{wt\% yield}}{\text{total conversion}} \times 100$

\*\* Conversion data corrected for 65/35 zeolite/binder

## Claims

1. A process for isomerizing an isomerization feed containing an aromatic $C_8$ mixture of ethylbenzene and xylene by which the para-xylene content is enhanced, said process comprising contacting said feed under conversion conditions with a catalyst comprising ZSM—23 zeolite having pores which are substantially unobstructed by silica.

2. A process as claimed in claim 1 wherein the zeolite is prepared from a non-gelling forming mixture containing amorphous precipitated silica as a silica source.

3. The process of claim 1 or claim 2 wherein said zeolite contains a Group VIII metal.

4. The process of claim 3 wherein said Group VIII metal is platinum.

5. The process of any preceding claim wherein said conversion conditions include a temperature of from 260°C to 538°C, a pressure of 0 to 1500 psig (100—10450 kPa) and a weight hourly space velocity of between 0.5 and 100.

## Patentansprüche

1. Verfahren zur Isomerisierung einer Isomerisationszufuhr, die eine aromatische $C_8$-Mischung von Ethylbenzol und Xylol enthält, wodurch der Gehalt an p-Xylol erhöht wird, wobei dieses Verfahren den Kontakt dieser Zuführ unter Umwandlungsbedingungen mit einem Katalysator umfaßt, der einen Zeolith ZSM—23 mit Poren umfaßt, die im wesentlichen nicht durch Siliziumdioxid blockiert sind.

2. Verfahren nach Anspruch 1, worin dieser Zeolith aus einer Mischung gebildet wird, die kein Gel bildet, die als Siliziumdioxidquelle amorph ausgefälltes Siliziumdioxid enthält.

3. Verfahren nach Anspruch 1 oder Anspruch 2, worin dieser Zeolith ein Metall der Gruppe VIII enthält.

4. Verfahren nach Anspruch 3, worin das Metall der Gruppe VIII Platin ist.

5. Verfahren nach einem der vorstehenden Ansprüche, worin die Umwandlungsbedingungen eine Temperatur von 260 bis 538°C, einen Druck von 0 bis 1500 psig (100 bis 10450 kPa) und eine stündliche Gewichts-Raumgeschwindigkeit von 0,5 und 100 umfassen.

## Revendications

1. Un procédé d'isomérisation d'une charge à isomériser contenant un mélange d'aromatiques en $C_8$ d'éthylbenzène et de xylène, par lequel il y a augmentation de la teneur en para-xylène, ce procédé consistant à mettre cette charge, dans des conditions de conversion, au contact d'un catalyseur comprenant une zéolite ZSM—23 dont les pores sont sensiblement non-obstrués par de la silice.

2. Un procédé selon la revendication 1, dans lequel la zéolite est préparée à partir d'un mélange exempt de gel contenant de la silice précipitée comme source de silice.

3. Un procédé selon la revendication 1 ou 2, dans lequel cette zéolite contient un métal du groupe VIII.

4. Un procédé selon la revendication 3, dans lequel ce métal du groupe VIII est le platine.

5. Un procédé selon l'une quelconque des revendications précédentes, dans lequel les conditions de conversion comprennent une température de 260 à 538°C, une pression de 100 à 10450 kPa (0 à 1500 psig) et une vitesse spatiale horaire pondérale comprise entre 0,5 et 100.